# EUROPÄISCHE PATENTSCHRIFT

(11) **EP 2 029 215 B1**
(45) Veröffentlichungstag und Bekanntmachung des Hinweises auf die Patenterteilung: **13.03.2019**
(21) Anmeldenummer: 07720156.4
(22) Anmeldetag: 22.05.2007
(51) Int. Cl.: A61M 25/10

(54) **MONORAIL-KATHETER**
MONORAIL CATHETER
CATHÉTER MONORAIL

(30) Priorität: 07.06.2006 CH 922062006
(43) Veröffentlichungstag der Anmeldung: 04.03.2009
(73) Patentinhaber: SIS Medical AG, 8500 Frauenfeld (CH)
(72) Erfinder: SCHWAGER, Michael, 8404 Winterthur (CH)
(74) Vertreter: Keller & Partner Patentanwälte AG
(86) Internationale Anmeldenummer: PCT/CH2007/000259
(87) Internationale Veröffentlichungsnummer: WO 2007/140634

(56) Entgegenhaltungen:
- US-A- 5 400 789
- US-A1- 2004 122 464
- US-A1- 2005 171 570

## Beschreibung

### Technisches Gebiet

Die Erfindung bezieht sich auf einen Monorail-Katheter mit einer einstückig ausgebildeten oder einer aufgesetzten Katheterspitze, welcher Monorail-Katheter ein- oder mehrschichtig als Ballonkatheter ausgeführt ist und ein Führungsdrahtlumen, sowie ein Zuführ-/Abführlumen zum Befüllen und Entleeren des Ballons aufweist. Weiter betrifft die Erfindung ein Verfahren zum Betrieb des Monorail-Katheters.

### Stand der Technik

Katheter sind Röhrchen oder Schläuche verschiedenen Durchmessers, zum Beispiel aus Kunststoff, Latex, Silikon, Metall oder Glas. Mit ihnen werden röhrenförmige, Körperflüssigkeiten führende Hohlorgane, nachfolgend kurz Organ(e) genannt, zum Beispiel Harnröhre, Speiseröhre, Gallengänge, Gefässe und blutführende Adern wie Herzarterien, von Mensch und/oder Tier, sondiert, durchstossen, entleert, gefüllt und/oder gespült. Dies geschieht aus diagnostischen (untersuchungsbedingten) oder therapeutischen (behandlungsbedingten) Gründen.

Es sind Katheter bekannt, welche einem Führungsdraht folgend in ein Organ eingeführt werden. Sind solche Katheter durch eine einzige Person bedienbar beziehungsweise in ein Organ einführ- und herausziehbar, werden sie vorliegend als Monorail-Katheter bezeichnet, auch in Verbindung mit einem Ballon, Ballone können mit einem flüssigen oder mit einem gasförmigen Medium gefüllt und entleert werden und dienen dazu, einen bestimmten Kathetertyp im betreffenden Organ eines Menschen/eines Tieres zu fixieren, dieses abzudichten und/oder aufzuweiten.

So wird in der US 2005/171570 A1 (Advanced Cardiovascular Systems) eine Vorrichtung zum schnellen Austausch von Dilatationskathetern beschrieben, die von einer Person bedient werden kann. Der Dilatationskatheter kann schnell ausgetauscht werden, weil der Führungsdraht ca. 10-15 cm hinter dem distalen Ende des Ballonkatheters aus dem eigentlichen Katheter hinausgeführt wird.

Heute übliche Monorail-Katheter in Verbindung mit einem Ballon weisen eine Wandstärke von etwa 20 µm auf. Sie bestehen beispielsweise aus einem Polyamid, Latex, Polyvinylchlorid (PVC) oder Silikon und werden bei einem Arbeitsdruck von etwa 10 bis 30 bar eingesetzt.

Eine zentrale Anforderung an einen Monorail-Katheter in Kombination mit einem Ballon ist ein möglichst kleiner Aussendurchmesser und möglichst kleine Wandstärken. Gleichzeitig müssen die Gleit- und Flexibilitätseigenschaften möglichst gut und die Reiss- und/oder Stauchgefahr während des Einführvorgangs klein sein. Die Kombination aus geringer Wandstärke bei gleichzeitig sehr guten Gleit- und Flexibilitätseigenschaften ermöglicht einen vergleichsweise geringen Widerstand beim Einführen des Katheters in das betreffende Organ. Dies auch deswegen, weil sich ein flexibler Katheter dem Verlauf eines Organs, in das er eingeführt wird, anpasst. Eine weitere wichtige Anforderung besteht darin, dass ein Ballon im eingeführten und befüllten Zustand trotz der dünnen Wandstärken und dem hohen Arbeitsdruck nicht platzen darf.

Die Anforderungen bezüglich den Gleit-/Flexibilitätseigenschaften sowie bezüglich den Reiss-/Staucheigenschaften gelten wenigstens in gleichem Masse auch für die (Monorail-)Katheterspitzen, welche nach dem Stand der Technik durch einen kleineren Aussendurchmesser im Bereich der Spitze einem Führungsdraht folgend das Einführen eines Monorail-Katheters in das betreffende Organ vorspurt und damit erleichtert. (Monorail-)Katheterspitzen können auch auf einen Katheter aufsetzbar ausgeformt sein. Aufsetzbare (Monorail-)Katheterspitzen erlauben eine von den restlichen Katheterteilen/- elementen unabhängige Herstellung, wodurch die genannten Anforderungen weiter optimiert werden können.

Ein wesentlicher Nachteil der gegenwärtigen Monorail-Katheter in Verbindung mit einem Ballon besteht darin, dass ein bestimmter Monorail-Kathetertyp aufgrund der genannten Materialanforderungen und der kleinen Aussenabmessungen nur einen sehr eingeschränkten Durchmesserbereich eines Organs abdichten/aufweiten kann, beispielsweise einen Durchmesserbereich von 1 bis 2 mm, von 2 bis 3 mm, oder von 3 bis 4 mm.

Ein weiterer Nachteil der gegenwärtigen Monorail-Katheter in Verbindung mit einem Ballon besteht darin, dass sie keine Möglichkeit zum Einbringen eines flüssigen und/der gasförmigen Mediums, vorteilhaft eine Lösung und/oder eine Stammzelle, vor den Monorail-Katheter beziehungsweise vor die Katheterspitze bieten. Aufgrund der vielfältigen Anforderungen aus Medizin und/oder Forschung ist dies aber oft wünschenswert.

### Darstellung der Erfindung

Aufgabe der Erfindung ist es, ein dem eingangs genannten technischen Gebiet zugehörenden Monorail-Katheter zu schaffen, welcher im Vergleich zu bisherigen Monorail-Kathetern
- zusätzlich das Einbringen eines flüssigen und/oder gasförmigen Mediums vor die Katheterspitze ermöglicht,
- und darüber hinaus ein Verfahren zum Betrieb des Monorail-Katheters ermöglicht, welches ein sicheren Abdichten gegenüber einem gegebenenfalls zurückfliessenden flüssigen und/oder gasförmigen Medium für einen grösseren Durchmesserbereich von Organen von Mensch und/oder Tier erlaubt als bisher,
- und ferner ein sanftes und effektives Einführen des Katheters ermöglicht, beispielsweise auch in sehr enge Gefässe und/oder durch Verengungen im Gefäss hindurch.

Die Lösung der Aufgabe ist durch die Merkmale des Anspruchs 1 definiert. Gemäss der Erfindung umfasst der Monorail-Katheter wenigstens ein freies Lumen zum Einbringen eines flüssigen und/oder gasförmigen Mediums. Das äusserste Lumen oder ein Aussenschaft weist am vorderen Ende eine nach vorne zulaufende Schräge auf. Spezielle und weiterbildende Ausführungsformen sind Gegenstand von abhängigen Patentansprüchen.

Durch das Einfügen des zusätzlichen, freien Lumens im Monorail-Katheter in Verbindung mit einem Ballon wird das Einbringen eines flüssigen und/oder gasförmigen Mediums, insbesondere eine Lösung und/oder eine Stammzelle, direkt vor die Spitze des Monorail-Katheters ermöglicht, was ein zentraler Vorteil gegenüber dem Stand der Technik darstellt. Sind in einer weiteren Variante statt eines zwei freie Lumen im Monorail-Katheter mit wenigstens einem Ballon angeordnet, können beispielsweise zwei unterschiedliche flüssige Medien direkt vor der Katheterspitze im entsprechenden Organ eingebracht beziehungswelse appliziert werden, die sich nicht bereits vor dem oder im Monorail-Katheter vermischen dürfen.

Die äussere Schicht des erfindungsgemässen Monorail-Katheters besteht vorteilhaft aus einem flexiblen, aber nur gering dehnbaren Werkstoff, vorzugsweise aus einem Polyamid, Nylon und/oder Polyester. Die innerste Schicht besteht aus einem Werkstoff hoher Druckfestigkeit, vorzugsweise aus High Density Polyethylen (HDPE), und eine allfällig vorhandene Bindezwischenschicht aus einem Werkstoff mit guten Adhäsionseigenschaften, vorzugsweise aus Plexar. Durch diese Anordnung werden die Anforderungen bezüglich den Gleit-/Flexibilitätseigenschaften sowie bezüglich den Reiss-/Stauchelgenschaften gegenüber dem Stand der Technik wenigstens beibehalten, allenfalls auch verbessert.

Der Durchmesser der Austrittsöffnung des freien Lumens beträgt im Bereich der Katheterspitze vorzugsweise 0,05 bis 0,45 mm, insbesondere etwa 0,35 mm. Vorzugsweise weist das freie Lumen über die gesamte Längsachse den selben Durchmesser wie an der Austrittsöffnung auf, was das Einführen des entsprechenden Mediums erleichtert.

In weiteren vorteilhaften Varianten ist der Monorail-Katheter mit einem über eine Katheter-Längsachse abschnittsweise konstanten oder veränderlichen, kreisförmigen, ovalen und/oder elliptischen Querschnitt ausgebildet.

Weitere Ausführungsformen des Monorail-Katheters zeichnen sich dadurch aus, dass wenigstens eine Schicht und/oder der Führungsdraht wenigstens teilweise gefärbt, reflektierend und/oder fluoreszierend ausgebildet ist und/oder eine röntgendichte Markierung eingebracht ist, vorzugsweise ein röntgendichter Markierungsring. Auf diese Weise ist es möglich, den Verlauf beziehungsweise die Position des Monorail-Katheters im Körper eines Menschen und/oder eines Tieres sowohl während des Einführens als auch im eingeführten Zustand, zum Beispiel mit einem Röntgen- oder einem Ultraschallgerät, nachzuvollziehen.

Der Führungsdraht selbst ragt beim Einführen über die Spitze des Monorail-Katheters hinaus und folgt dank seines geringen Widerstands infolge des kleinen Durchmessers und dessen Flexibilität dem Verlauf des Organs. Dabei folgt die Katheterspitze beziehungsweise der Monorail-Katheter dem Führungsdraht und schmiegt sich dem Verlauf des Organs an. Der Führungsdraht ist aus gut gleitfähigem, flexiblem und gleichzeitig zähem Material gefertigt, vorzugsweise aus rostfreiem Stahl, Aluminium oder aus Kunststoffen mit hoher mechanischer Festigkeit, zum Beispiel PVC, einem Polyamid oder einem Polyester.

In einer vorteilhaften Variante ist nur eine innerste Schicht des Monorail-Katheters beziehungsweise des Führungsdrahtlumens gegenüber dem Führungsdraht gleitfähig ausgebildet. Die vorliegende Erfindung funktioniert grundsätzlich auch ohne einen Führungsdraht, zum Beispiel, wenn der Monorail-Katheter in ein Organ eingeführt wird, welches vergleichsweise kurz und/oder geradlinig ist.

Bevorzugt ist die röntgendichte Markierung innerhalb eines Lumens des Katheters angebracht. Dadurch hat die Markierung keinen Einfluss auf das Profil des zusammengefalteten Ballons, was die Einführbarkeit des Katheters deutlich verbessert, da keine seitlich vorstehenden Widerstandsflächen vorliegen.

Von Vorteil verläuft innerhalb des Lumens, welches die röntgendichte Markierung beinhaltet, ein weiteres Lumen. Damit kann die röntgendichte Markierung bevorzugt an diesem weiteren Lumen befestigt werden. Insbesondere lässt sich die röntgendichte Markierung am Lumen festklemmen oder stoffschlüssig mit diesem verbinden. Dies vereinfacht die Herstellung eines derartigen Monorail-Katheters und stellt zudem sicher, dass die röntgendichte Markierung auch bei einer starken Beanspruchung an einer genau definierten Lage fixiert bleibt.

In einer vorteilhaften Variante weist eine äussere Begrenzung der röntgendichten Markierung in Längsrichtung des Lumens einen ovalen oder elliptischen Querschnitt auf. Die Querschnittsfläche der äusseren Begrenzung ist dabei kleiner, als ein innerer Querschnitt des Lumens, welches die röntgendichte Markierung beinhaltet. Damit wird erreicht, dass das Lumen, welches die röntgendichte Markierung beinhaltet, für Flüssigkeiten und/oder Gase entlang seiner gesamten Länge durchlässig bleibt und somit beispielsweise als Zuführ- oder Abführlumen eingesetzt werden kann. Überraschenderweise wurde gefunden, dass ein ovaler oder elliptischer Querschnitt der äusseren Begrenzung der Markierung dabei ein optimales Strömungsverhalten von Flüssigkeiten und/oder Gasen im Bereich der Markierung ergibt. Verglichen mit einer kreisrunden Querschnittsfläche, wurde bei den ovalen oder elliptischen Querschnittsflächen ein grösserer Durchfluss durch das identische Lumen erreicht.

In einer weiteren vorteilhaften Variante weist ein äusserstes Lumen oder ein Aussenschaft an einem vorderen Ende, welches insbesondere innerhalb des Ballons liegt, eine nach vorne zulaufende Schräge auf, bzw. die Endfläche des Lumens bildet eine Ebene im 45°-Winkel zur Längsachse des Lumens. Eine derartige Ausformung erlaubt ein sanftes und effektives Einführen des Katheters beispielsweise auch in sehr enge Gefässe und/oder durch Verengungen im Gefäss hindurch. Durch die Schräge werden dabei die Gefässwände langsam und kontinuierlich aufgeweitet, was besonders schonend für das Gefäss ist.

Besonders vorteilhaft ist es zudem, wenn das äusserste Lumen oder der Aussenschaft am vorderen Ende in einem Mantelbereich gewindeartig ausgeformt ist. Damit können enge Stellen in Gefässen beim Einführen des Katheters noch besser passiert werden. Dies ist darauf zurückzuführen, dass die gewindeartige Ausformung den Gleitwiderstand und das Biegeverhalten bzw. die Flexibilität des äussersten Lumens im vorderen Bereich verbessert. Insbesondere wird das Lumen flexibler aber gleichzeitig auch knickstabiler, was ein Einführen des Katheters mit dem darüber liegenden und zusammengefalteten Ballon insgesamt verbessert.

Die Katheterspitze des Monorail-Katheters ist vorteilhaft nach vorne schräg zulaufend ausgebildet, wobei ein Aussendurchmesser an der Vorderseite der Katheterspitze vorzugsweise 0,35 bis 0,5 mm, insbesondere etwa 0,45 mm, und/oder ein Aussendurchmesser an der Rückseite der Katheterspitze vorzugsweise 0,45 bis 0,6 mm, insbesondere etwa 0,5 mm, beträgt.

Der Monorail-Katheter zeichnet sich in weiteren vorteilhaften Varianten dadurch aus, dass die Katheterspitze eine Länge von 0,5 bis 10 mm, insbesondere 3 bis 5 mm, und/oder der Monorail-Katheter eine Gesamtlänge von 800 bis 2'000 mm, insbesondere 1'200 bis 1'600 mm, aufweist.

In einer weiteren Variante ist der Monorail-Katheter im Verbindungsbereich und/oder wenigstens an einem Ballonende vorzugsweise weich und einschichtig ausgeführt, um eine stoff- und/oder kraftschlüssige Verbindung zwischen dem Monorail-Katheter und der aufsetzbaren Katheterspitze beziehungsweise das Verbinden des Ballons mit wenigstens einem Lumen zu unterstützen. Auch eine einstückig ausgebildete Katheterspitze ist in einer vorteilhaften Variante ganz oder teilweise weich und/oder einschichtig ausgeführt.

Weiterhin ist im Verbindungsbereich vorteilhaft
- der Monorail-Katheter stirnflächig zur Katheterspitze verbindbar ausgeformt, und/oder
- der Monorail-Katheter in die Katheterspitze und/oder die Katheterspitze in den Monorail-Katheter einschiebbar.

Eine weitere Ausführungsform des erfindungsgemässen Monorail-Katheters zeichnet sich dadurch aus, dass der Verbindungsbereich zur aufsetzbaren Katheterspitze verschweissbar, verklebbar, pressbar und/oder pressschmelzbar ausgeführt ist.

Eine weitere vorteilhafte Ausführungsform des Monorail-Katheters zeichnet sich dadurch aus, dass eine Ballonoberfläche wenigstens partiell eine parallele und/ oder kreuzweise Profilierung oder Texturierung aufweist, welche vorzugsweise genoppt und/oder gerillt ist. Der Ballon weist des Weiteren eine Wandstärke von 1 bis 10 µm, vorzugsweise etwa 3 µm, auf, und besteht vorzugsweise aus Pebax, was ein Co-Polymer darstellt, oder aus Nylon.

Die Aufgabe bezüglich des Verfahrens zum Betrieb des Monorail-Katheters wird erfindungsgemäss dadurch gelöst, dass der Ballon oder mehrere Ballons des gleichen Durchmessers mit 0,5 bis 2 bar beaufschlagt wird. Vorzugsweise wird der Ballon mit etwa 1 bar beaufschlagt.

Im Vergleich zum Stand der Technik wird mit der vorliegenden Erfindung auf die Funktion des Aufweitens eines Organs verzichtet. Der Ballon des erfindungsgemässen Monorail-Katheters wird aus diesem Grund mit einem wesentlich geringeren Druck beaufschlagt, konkret mit vorzugsweise etwa 1 bar, was zum Beispiel folgende Vorteile ergibt:
- Der Druck ist gegenüber dem Stand der Technik wesentlich kleiner; das betreffende Organ wird geschont, wodurch dieses keine Schädigung beziehungsweise kein Trauma erleidet.
- 1 bar ist ein Druck, welcher in (Forschungs-)Laboratorien und Medizinalbereichen üblicherweise standardmässig verfügbar ist.
- Es müssen nicht, wie heute oft üblich, für bestimmte Durchmesserbereiche entsprechenden Drücke eingestellt werden. Dieser Umstand entlastet insbesondere das Pflegepersonal bei deren Tätigkeiten, weil es nicht mehr an das Einstellen eines bestimmten Druckes in Verbindung mit einem bestimmten Monorail-Kathetertyp denken muss. Dies erhöht letztlich auch die Sicherheit bei operativen Eingriffen.

In einer weiteren vorteilhaften Variante zum Betrieb des Monorail-Katheters dichtet derselbe Ballon Organe von Mensch und/oder Tier, vorzugsweise Herzkranzgefässe, in einem Durchmesserbereich von vorzugsweise 1,5 bis 5,5 mm ab.

Überraschend erlaubt der tiefe Arbeitsdruck von vorzugsweise etwa 1 bar auch bei einem vergleichsweise engen Organ ein sicheres Abdichten. Weist ein Organ zum Beispiel einen Innendurchmesser von 2 mm auf, so dichtet der Ballon des erfindungsgemässen Monorail-Katheters trotz des vermeintlich überflüssigen Ballonmaterials durch das Anschmiegen von dessen Falten gegenüber der Innenwand des Organs sicher. Umgekehrt wird auch ein vergleichsweise weites Organ mit beispielsweise 5 mm Innendurchmesser sicher abgedichtet, wobei kaum noch Falten vorhanden sind.

Somit lässt sich mit einem einzigen Monorail-Kathetertyp in Ballonbauweise ein wesentlich grösserer Durchmesserbereich abdecken als mit bekannten Monorail-Kathetern, was beachtliche Vorteile mit sich bringt, zum Beispiel:
- Geringere oder keine Verwechslungsgefahr beim Handhaben des Monorail-Katheters, da im Idealfall nur ein Monorail-Kathetertyp zum Abdichten eines Organs vorgehalten werden muss.
- Entsprechend werden unnötige Eingriffe durch das Einführen von falschen beziehungsweise ungeeigneten Kathetern verringert oder ganz vermieden.
- Insgesamt sind weniger und im Idealfall keine Vorabklärungen zur Bestimmung eines geeigneten Monorail-Kathetertyps notwendig.
- Es fallen geringere Anschaffungs-, Unterhalts- und Lagerhaltungskosten an, da auf bestimmte Monorail-Kathetertypen verzichtet werden kann.

Der erfindungsgemässe Monorail-Katheter stellt deshalb im Vergleich zum Stand der Technik eine wesentliche Weiterentwicklung dar und ist als wegweisende Innovation im Dienste der Medizin für Mensch und Tier zu sehen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine teilweise aufgeschnittene Seitenansicht eines Monorail-Katheters mit einer einstückig ausgebildeten Spitze in einem Herzkranzgefäss,
- Fig. 2: einen Monorail-Katheter gemäss Fig. 1 mit einer aufgesetzten Spitze,
- Fig. 3: eine Ansicht von vorne auf den Monorail-Katheter gemäss Fig. 2,
- Fig. 4: eine Ansicht von vorne auf den Monorail-Katheter gemäss Fig. 2 mit einem zweiten freien Lumen,
- Fig. 5: eine Ansicht von vorne auf den Monorail-Katheter gemäss Fig. 2 ohne Führungsdrahtlumen,
- Fig. 6: die abgeschrägte Spitze des Monorail-Katheters gemäss Fig. 1 und 2, und
- Fig. 7: eine Variante von Fig. 6,
- Fig. 8: eine aufgeschnittene Seitenansicht eines Monorail-Katheters mit einer röntgendichten Markierung im Zuführ-/Abführlumen,
- Fig. 9: ein Querschnitt durch den Monorail-Katheter aus Fig. 8 entlang der Linie A - B,
- Fig. 10: eine Detailansicht des hintern Bereichs eines Monorail-Katheters mit einem in der Ballonhülle schräg zulaufenden Zuführ-/Abführlumen.

Grundsätzlich sind in den Figuren gleiche Teile mit gleichen Bezugszeichen versehen.

### Wege zur Ausführung der Erfindung

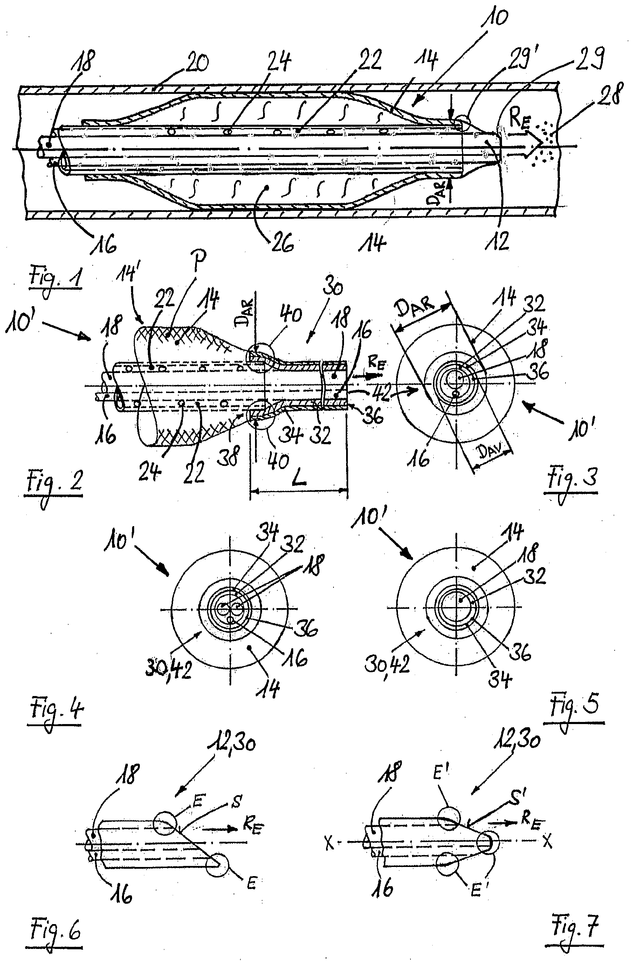

**Fig. 1** zeigt eine teilweise aufgeschnittene Seitenansicht eines Monorail-Katheters 10 mit einer einstückig ausgebildeten Katheterspitze 12, wobei der Monorail-Katheter 10 in Verbindung mit einem einschichtigen Ballon 14 ausgeführt ist und ein Führungsdrahtlumen 16 sowie ein freies Lumen 18 umfasst. Auf die Darstellung eines Führungsdrahtes ist verzichtet worden, er ist allgemein bekannt. Der einschichtige Ballon 14 ist nach dem Einführen des Monorail-Katheters 10 in das Herzkranzgefäss 20 über ein Zuführ-/Abführlumen 22 beziehungsweise dessen Ein-/Austrittsöffnung 24 mit einem Gas 26 gefüllt, hat sich infolgedessen aufgeweitet und steht unter einem Druck von etwa 1 bar. Der Ballon 14 dichtet auf dieses Weise das Herzkranzgefäss 20 aussergewöhnlich schonend ab, worauf ein flüssiges und/oder gasförmiges Medium 28 in Einführrichtung R_{E} aus der Vorderseite 29 der einstückig ausgebildeten Katheterspitze 12 in das Herzkranzgefäss 20 eingeführt werden kann. Auf die Darstellung von abdichtenden Ballonfalten ist der Übersichtlichkeit wegen verzichtet worden.

Der Monorail-Katheter 10' weist im Bereich der Oberfläche 14', welche das Herzkranzgefäss 20 im aufgeweiteten Zustand an der Gefässinnenseite berührt, eine kreuzweise Profilierung P auf. Die Profilierung P unterstützt beim Einbringen des flüssigen und/oder gasförmigen Mediums 28 den Halt gegenüber einer dabei entstehenden, der Einführrichtung R_{E} entgegen wirkenden Druckkraft. In anderen nicht dargestellten Varianten weist die Ballonoberfläche 14' auch parallele und/oder kreuzweise ausgebildete Noppen oder Rillen auf.

Nach dem entsprechenden Eingriff wird das Gas 26 über das Zuführ-/Abführlumen 22 beziehungsweise dessen Ein-/Austrittsöffnung 24 abgeführt, worauf der Ballon 14 zusammensackt und der Monorail-Katheter 10 in an sich bekannter Weise aus dem Herzkranzgefäss 20 entnommen werden kann. Der einschichtige Ballon 14 ist im Bereich der Rückseite 29' der einstückig ausgebildeten Katheterspitze 12 mit dem Zuführ-/Abführlumen 22 verschweisst, wobei der Aussendurchmesser D_{AR} in diesem Bereich etwa 0,5mm beträgt. Weiterhin beträgt die Gesamtlänge des Monorail-Katheters 10 im vorliegenden Fall etwa 1'400 mm.

**Fig. 2** zeigt eine weitere, bevorzugte, Ausführungsvariante des ansatzweise dargestellten Monorail-Katheters 10' mit einer aufgesetzten Katheterspitze 30, welche dreischichtig ausgeführt ist und eine Länge L von etwa 4 mm aufweist. Die aufsetzbare Katheterspitze 30 ist in einem separaten Gesuch mit demselben Einreichungsdatum wie die vorliegende Erfindung zum Patent angemeldet.

Die aufgesetzte Katheterspitze 30 weist als eine Besonderheit zwei zylindrisch ineinander liegende Schichten 32, 34 auf, welche durch eine dünne Bindezwischenschicht 36 getrennt sind. Die Bindezwischenschicht 36 ist der Übersichtlichkeit wegen nur als einfacher Strich dargestellt und ist etwa 2 µm dick. Wie erwähnt besteht dabei die innere Schicht 32 aus einem Werkstoff hoher Druckfestigkeit, in der dargestellten Variante aus High Density Polyethylen (HDPE), die Bindezwischenschicht 36 aus einem Werkstoff mit guten Adhäsionseigenschaften, im Beispiel aus Plexar, und die äussere Schicht 34 aus einem flexiblen, aber nur gering dehnbaren Werkstoff, im Beispiel aus Polyamid (PA). Weiter ist in Fig. 2 ebenfalls ein Führungsdrahtlumen 16, ein Zuführ-/Abführlumen 22 sowie ein freies Lumen 18 dargestellt.

Die Rückseite 38 der aufgesetzten Katheterspitze 30 ist in einem Verbindungsbereich 40 auf dem Monorail-Katheter 10' aufgesetzt, nach einer nicht dargestellten Variante eingesetzt. Die aufgesetzte Katheterspitze 30 ist mit dem Monorail-Katheter 10' unter Druck verschweisst (pressgeschmolzen). Der Aussendurchmesser D_{AR} an der Rückseite 38 der aufgesetzten Katheterspitze 30 beträgt nach dem Aufsetzen etwa 0,5 mm

**Fig. 3** zeigt eine Ansicht auf die Vorderseite 42 der aufgesetzten Katheterspitze 30 des Monorail-Katheters 10' gemäss Fig. 2. Dabei wird das Führungsdrahtlumen 16 und das freie Lumen 18 verdeutlicht. Der Aussendurchmesser D_{AV} an der Vorderseite 42 der aufgesetzten Katheterspitze 30 beträgt etwa 0,45 mm

**Fig. 4** und **Fig. 5** zeigen als Ausführungsvarianten ebenfalls eine Ansicht auf die Vorderseite 42 der aufgesetzten Katheterspitze 30 gemäss Fig. 2. Im Unterschied zu Fig. 2 weist die Fig. 4 hierbei ein zweites freies Lumen 18 auf, wodurch zwei unterschiedliche Medien 28 direkt vor der aufgesetzten Katheterspitze 30 in das betreffende Organ eingeführt werden können. In Fig. 5 wird im Unterschied zu den Fig. 1 bis 4 und auf ein Führungsdrahtlumen 16 verzichtet. Wie erwähnt ist diese Variante besonders gut geeignet, wenn der Monorail-Katheter 10, 10' in ein Organ eingeführt wird, welches vergleichsweise kurz und/oder geradlinig ist. Entsprechend grösser ist nun der Querschnitt des freien Lumens 18 gestaltet. Das freie Lumen 18 kann in dieser Ausführungsform beispielsweise vorteilhaft zum Einführen einer Stammzelle genutzt werden.

Die Fig. 3 bis 5 zeigen Ausführungsvarianten der aufgesetzten Katheterspitze 30, welche vorteilhaft auch mit der in Fig. 1 dargestellten einstückig ausgebildeten Katheterspitzen 12 umgesetzt sind. Die angedeuteten Schichten 32, 34, 36 der aufgesetzten Katheterspitze 30 müssen dabei lediglich als eine einzelne Schicht der einstückig ausgebildeten Katheterspitze 12 angesehen werden

**Fig. 6** zeigt die Vorderseite 29, 42 der Katheterspitze 12, 30 des Monorail-Katheters 10, 10'. Die dargestellte Variante weist eine nach vorne einseitig zulaufende Schräge S auf, so dass die Endfläche des Lumens eine Ebene im 45°-Winkel zur Längsachse des Lumens bildet. Diese Ausführungsform ist dann besonders vorteilhaft, wenn der Monorail-Katheter 10, 10' in ein Organ mit kleinem Querschnitt eingeführt werden muss. Die Schräge S weitet das Organ während des Einführvorgangs kontinuierlich auf. Um das betreffende Organ vor Verletzungen zu schützen, ist die Schräge S an deren Enden E abgerundet, insbesondere am spitzen Ende.

**Fig. 7** stellt als Variante der Fig. 6 eine Schräge S' dar, welche beidseitig, symmetrisch zur Längsmittelachse X-X, nach vorne zuläuft. Auch die Schräge S' ist an deren Enden E' abgerundet.

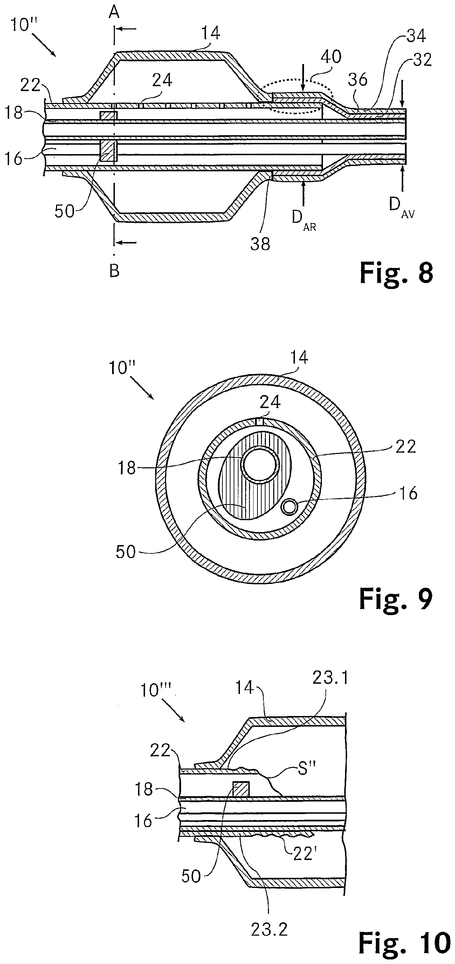

In **Fig. 8** ist ein weiterer erfindungsgemässer Monorail-Katheter 10" im Längsschnitt dargestellt. Im Unterschied zum Katheter 10', welcher in Fig. 2 beschrieben ist, weist der Monorail-Katheter 10" innerhalb des Zuführ-/Abführlumens 22 in einem hinteren Bereich, des Ballons 14 eine röntgendichte Markierung in Form eines Platinrings 50 auf. Der Innendurchmesser des kreisrunden Zuführ-/Abführlumens 22 misst 0.62 mm.

**Fig. 9** zeigt einen Querschnitt entlang der Linie A - B in Fig. 8. Daraus ist ersichtlich, dass der Platinring 50 in einer Längsrichtung des im Querschnitt kreisrunden Zuführ-/Abführlumens 22 eine im Querschnitt ovale bzw. elliptische äussere Begrenzung aufweist. Ungefähr in einem der Brennpunkte der elliptischen Begrenzung weist der Platinring 50 eine zylindrische Bohrung mit einem Durchmesser von 0.54 mm auf, was dem Aussendurchmesser des freien Lumens 18 entspricht. Das freie Lumen 18, welches ebenfalls innerhalb des Zuführ-/Abführlumens 22 verläuft, führt direkt durch die Bohrung des Platinrings 50 und dient als Verankerung für diesen. Der Platinring 50 wurde bei der Herstellung des Monorail-Katheters 10" von aussen zusammengepresst, sodass der Platinring 50 kraftschlüssig auf dem freien Lumen 18 fixiert und festgeklemmt ist. Das Führungsdrahtlumen 16, führt seitlich neben dem Platinring 50 vorbei. Entlang des gesamten Umfangs des Platinrings 50 besteht zwischen dem Platinring 50 und dem Zuführ-/Abführlumen 22 ein freier Durchgang für Flüssigkeiten oder Gase.

Fig. 10 zeigt eine weitere Variante eines Monorail-Katheters 10"'. Ein Zuführ-/Abführlumen 22 mit einem Innendurchmesser von 0.62 mm reicht dabei im Gegensatz zu den vorherigen Ausführungsbeispielen lediglich bis in den hinteren Bereich des Ballons 14. Im Zuführ-/Abführlumen 22 verläuft in einem unteren Bereich an der Innenseite ein freies Lumen 18. Im freien Lumen 18 verläuft wiederum in einem unteren Bereich ein Führungsdrahtlumen 16. Das freie Lumen 18, sowie das Führungsdrahtlumen 16 reichen bis in die nicht dargestellten vorderen Bereiche des Katheters 10"'. Die Spitze des Katheters 10"' ist dabei im Wesentlichen analog zu den Ausführungsbeispielen der Fig. 8 ausgeführt. Das Zuführ-/Abführlumen 22 weist als Besonderheit eine nach vorne einseitig zulaufende Schräge S" auf, sodass die Endfläche des Zuführ-/Abführlumens 22 eine Ebene im 45°-Winkel zur Längsachse des Zuführ-/Abführlumens 22 bildet. Damit ist der untere Mantelbereich 23.2 des Zuführ-/Abführlumens 22 länger ausgebildet als der gegenüberliegende obere Mantelbereich 23.1. Das vordere Ende des Zuführ-/Abführlumens 22 ist des Weiteren als Aussengewinde 22' ausgeformt. Wie aus der Fig. 10 ersichtlich ist, ist im Zuführ-/Abführlumen 22 ebenfalls ein im Querschnitt ovaler Platinring 50 angebracht, welcher auf das freie Lumen 18 geklemmt ist. Das Zuführ-/Abführlumen 22 ist im Bereich der Schräge S" an seinem vorderen Ende offen und kommuniziert mit dem Ballon 14, welcher seinem hinteren Ende das Zuführ-/Abführlumen 22 vollständig umgibt und mit diesem verschweisst ist. Am vorderen Ende ist der Ballon 14 fluiddicht um das freie Lumen 18 herum verschweisst.

Alle Ausführungsvarianten sind im Bereich der Schräge S, S', S" der Katheterspitze 12, 30 und/oder im Verbindungsbereich 40 zu einer aufsetzbaren Katheterspitze 30 angeschmolzen. Ein Anschmelzen dieser Stellen bewirkt einen aussergewöhnlich gleichmässigen, glatten Übergang zwischen den unterschiedlichen Querschnitten und vermindert durch eine verbesserte Anschmiegbarkeit der Katheterspitze 10, 10' den Einführwiderstand in ein Organ.

Zusammenfassend ist festzustellen, dass ein neuartiger Monorail-Katheter entwickelt wurde, welcher sich sowohl durch eine verbesserte Einführbarkeit als auch durch eine hohe Funktionalität auszeichnet.

## Patentansprüche

1. Monorail-Katheter (10, 10', 10", 10"') mit einer einstückig ausgebildeten (12) oder einer aufgesetzten Katheterspitze (30), welcher Monorail-Katheter (10, 10', 10", 10'") ein-oder mehrschichtig als Ballonkatheter in Verbindung mit einem Ballon (14) ausgeführt ist und wenigstens ein Führungsdrahtlumen (16), sowie ein Zuführ-/Abführlumen (22) zum Befüllen und Entleeren des Ballons aufweist, und der Monorail-Katheter (10, 10', 10", 10'") wenigstens ein zusätzliches freies Lumen (18) zum Einbringen eines flüssigen und/oder gasförmigen Mediums umfasst, **dadurch gekennzeichnet, dass** ein äusserstes Lumen oder ein Aussenschaft (22) an einem vorderen Ende, welches innerhalb des Ballons (14) liegt, eine nach vorne zulaufende Schräge (S") aufweist.

2. Monorail-Katheter (10, 10', 10", 10'") nach Anspruch 1, **dadurch gekennzeichnet, dass** eine äussere Schicht (34) aus einem flexiblen, aber nur gering dehnbaren Werkstoff besteht, vorzugsweise aus Polyamid, Nylon und/oder Polyester.

3. Monorail-Katheter (10, 10', 10", 10'") nach Anspruch 1 oder 2, **dadurch gekennzeichnet, dass** die Austrittsöffnung des freien Lumens (18) im Bereich der Katheterspitze (12, 30) einen Durchmesser von 0,05 bis 0,45 mm aufweist, insbesondere etwa 0,35 mm.

4. Monorail-Katheter (10, 10', 10", 10'") nach einem der Ansprüche 1 bis 3, **dadurch gekennzeichnet, dass** er mit einem über die Katheter-Längsachse (x-x) abschnittsweise konstanten oder veränderlichen, kreisförmigen, ovalen und/ oder elliptischen Querschnitt ausgebildet ist.

5. Monorail-Katheter (10, 10', 10", 10'") nach einem der Ansprüche 1 bis 4, **dadurch gekennzeichnet, dass** wenigstens eine Schicht des Monorail-Katheters (10, 10', 10", 10'") und/oder der Führungsdraht wenigstens teilweise gefärbt, reflektierend und/oder fluoreszierend ausgebildet ist und/oder eine röntgendichte Markierung aufgebracht ist, vorzugsweise ein röntgendichter Markierungsring.

6. Monorail-Katheter (10, 10', 10", 10"') nach Anspruch 5, **dadurch gekennzeichnet, dass** die röntgendichte Markierung innerhalb eines der genannten Lumen (16,18, 22) des Katheters (10, 10', 10", 10'") angebracht ist.

7. Monorail-Katheter (10, 10', 10", 10'") nach Anspruch 6, **dadurch gekennzeichnet, dass** innerhalb des Lumens, welches die röntgendichte Markierung beinhaltet, ein weiteres Lumen verläuft, wobei die röntgendichte Markierung bevorzugt an diesem weiteren Lumen befestigt, insbesondere festgeklemmt, ist.

8. Monorail-Katheter (10, 10', 10", 10'") nach einem der Ansprüche 6 bis 7, **dadurch gekennzeichnet, dass** eine äussere Begrenzung der röntgendichten Markierung senkrecht zur Längsrichtung des Lumens einen ovalen oder elliptischen Querschnitt aufweist, welcher kleiner ist als ein innerer Querschnitt des Lumens, welches die röntgendichte Markierung beinhaltet.

9. Monorail-Katheter (10, 10', 10", 10'") nach Anspruch 1, **dadurch gekennzeichnet, dass** das äusserste Lumen oder der Aussenschaft (22) am vorderen Ende in einem Mantelbereich (22.1, 22.2) gewindeartig ausgeformt ist.

10. Monorail-Katheter (10, 10', 10", 10'") nach einem der Ansprüche 1 bis 9, **dadurch gekennzeichnet, dass** er im Bereich der Katheterspitze (12, 30) nach vorne schräg zulaufend ausgebildet ist, wobei ein Aussendurchmesser (D_{AV}) an der Vorderseite (29, 42) der Katheterspitze (12, 30) vorzugsweise 0,35 bis 0,5 mm, insbesondere etwa 0,45 mm, und/oder ein Aussendurchmesser (D_{AR}) an der Rückseite (29', 38) der Katheterspitze (12, 30) vorzugsweise 0,45 bis 0,6 mm, insbesondere etwa 0,5 mm, beträgt.

11. Monorail-Katheter (10, 10', 10", 10'") nach einem der Ansprüche 1 bis 10, **dadurch gekennzeichnet, dass** die Katheterspitze (12, 30) eine Länge (L) von 0,5 bis 10 mm, insbesondere 3 bis 5 mm, und/oder der Monorail-Katheter (10, 10', 10", 10'") eine Gesamtlänge von 800 bis 2'000 mm, insbesondere 1'200 bis 1'600 mm, aufweist.

12. Monorail-Katheter (10, 10', 10", 10'") nach einem der Ansprüche 1 bis 11, **dadurch gekennzeichnet, dass** ein Verbindungsbereich (40) zu einer aufsetzbaren Katheterspitze (30) und/oder eine Schräge (S) der einstückig ausgebildeten (12) oder der aufgesetzten Katheterspitze (30) angeschmolzen ist, vorzugsweise an einem oder an beiden Enden (E, E') der Katheterspitze (12, 30).

13. Monorail-Katheter (10, 10', 10", 10"') nach Anspruch 12, **dadurch gekennzeichnet, dass** der Monorail-Katheter (10, 10', 10", 10'") im Verbindungsbereich (40) und/oder wenigstens an einem Ballonende vorzugsweise weich und einschichtig ausgeführt ist.

14. Monorail-Katheter (10, 10', 10", 10'") nach Anspruch 12 oder 13, **dadurch gekennzeichnet, dass** im Verbindungsbereich (40) der Monorail-Katheter (10, 10', 10", 10'") stirnflächig zur Katheterspitze (12, 30) verbindbar ausgeformt ist, und/oder der Monorail-Katheter (10, 10', 10", 10'") in die Katheterspitze (12, 30) und/oder die Katheterspitze (12, 30) in den Monorail-Katheter (10, 10', 10", 10'") einschiebbar ist.

15. Monorail-Katheter (10, 10', 10", 10'") nach einem der Ansprüche 1 bis 14, **dadurch gekennzeichnet, dass** eine Ballonoberfläche (14') wenigstens partiell eine parallele und/oder kreuzweise Profilierung (P) oder Texturierung aufweist, welche vorzugsweise genoppt und/oder gerillt ist.

16. Verfahren zum Betrieb des Monorail-Katheters (10, 10', 10", 10'") nach einem der Ansprüche 1 bis 15, **dadurch gekennzeichnet, dass** der Ballon (14) oder mehrere Ballons (14) des gleichen Durchmessers mit 0,5 bis 2 bar beaufschlagt wird.

17. Verfahren zum Betrieb des Monorail-Katheters (10, 10', 10", 10'") nach Anspruch 16, **dadurch gekennzeichnet, dass** der Ballon (14) vorzugsweise mit 1 bar beaufschlagt wird.

18. Verfahren zum Betrieb des Monorail-Katheters (10, 10', 10", 10'") nach Anspruch 16 oder 17, **dadurch gekennzeichnet, dass** derselbe Ballon (14) Organe von Mensch und/oder Tier, vorzugsweise Herzkranzgefässe (20), in einem Durchmesserbereich von vorzugsweise 1,5 bis 5,5 mm abdichten kann.

## Claims

1. Monorail catheter (10, 10', 10", 10"') having an integrally formed (12) or attached catheter tip (30), which monorail catheter (10, 10', 10", 10"') is embodied in one or more layers as a balloon catheter in conjunction with a balloon (14) and has at least one guidewire lumen (16), and a delivery/discharge lumen (22) for filling and emptying the balloon, and the monorail catheter (10, 10', 10", 10"') comprises at least one additional free lumen (18) for introducing a liquid and/or gaseous medium, **characterized in that** an outermost lumen or an outer shank (22) has a forwardly tapering slope (S") at a front end, which is located within the balloon (14).

2. Monorail catheter (10, 10', 10", 10"') according to Claim 1, **characterized in that** an outer layer (34) consists of a flexible, but only slightly extensible material, preferably of polyamide, nylon and/or polyester.

3. Monorail catheter (10, 10', 10", 10"') according to Claim 1 or 2, **characterized in that** the outlet opening of the free lumen (18) has a diameter of 0.05 to 0.45 mm, in particular around 0.35 mm, in the region of the catheter tip (12, 30).

4. Monorail catheter (10, 10', 10", 10"') according to one of Claims 1 to 3, **characterized in that** it is formed with a circular, oval and/or elliptical cross section that is sectionally constant or variable along the catheter longitudinal axis (x-x).

5. Monorail catheter (10, 10', 10", 10"') according to one of Claims 1 to 4, **characterized in that** at least one layer of the monorail catheter (10, 10', 10", 10"') and/or of the guidewire is formed at least partially in a coloured, reflective and/or fluorescent manner and/or a radiopaque marking is applied, preferably a radiopaque marking ring.

6. Monorail catheter (10, 10', 10", 10"') according to Claim 5, **characterized in that** the radiopaque marking is applied within one of said lumina (16, 18, 22) of the catheter (10, 10', 10", 10'").

7. Monorail catheter (10, 10', 10", 10'") according to Claim 6, **characterized in that**, within the lumen which contains the radiopaque marking, a further lumen extends, wherein the radiopaque marking is fastened, in particular clamped, preferably to this further lumen.

8. Monorail catheter (10, 10', 10", 10'") according to either of Claims 6 and 7, **characterized in that** an outer boundary of the radiopaque marking has an oval or elliptical cross section perpendicularly to the longitudinal direction of the lumen, said cross section being smaller than an internal cross section of the lumen which contains the radiopaque marking.

9. Monorail catheter (10, 10', 10", 10'") according to Claim 1, **characterized in that** the outermost lumen or the outer shank (22) is formed in a threadlike manner in a lateral region (22.1, 22.2) at the front end.

10. Monorail catheter (10, 10', 10", 10"') according to one of Claims 1 to 9, **characterized in that** it is formed in a forwardly tapering manner in the region of the catheter tip (12, 30), wherein an outside diameter (D_{AV}) at the front side (29, 42) of the catheter tip (12, 30) is preferably 0.35 to 0.5 mm, in particular around 0.45 mm, and/or an outside diameter (D_{AR}) at the rear side (29', 38) of the catheter tip (12, 30) is preferably 0.45 to 0.6 mm, in particular around 0.5 mm.

11. Monorail catheter (10, 10', 10", 10"') according to one of Claims 1 to 10, **characterized in that** the catheter tip (12, 30) has a length (L) of 0.5 to 10 mm, in particular 3 to 5 mm, and/or the monorail catheter (10, 10', 10", 10"') has a total length of 800 to 2000 mm, in particular 1200 to 1600 mm.

12. Monorail catheter (10, 10', 10", 10"') according to one of Claims 1 to 11, **characterized in that** a connecting region (40) to an attachable catheter tip (30) and/or a slope (S) of the integrally formed (12) or attached catheter tip (30) is fused, preferably at one or both ends (E, E') of the catheter tip (12, 30).

13. Monorail catheter (10, 10', 10", 10"') according to Claim 12, **characterized in that** the monorail catheter (10, 10', 10", 10"') is embodied preferably soft and in one layer in the connecting region (40) and/or at least at one balloon end.

14. Monorail catheter (10, 10', 10", 10"') according to Claim 12 or 13, **characterized in that**, in the connecting region (40), the monorail catheter (10, 10', 10", 10"') is designed to be connectable to the catheter tip (12, 30) at the end face, and/or the monorail catheter (10, 10', 10", 10"') is introducible into the catheter tip (12, 30) and/or the catheter tip (12, 30) is introducible into the monorail catheter (10, 10', 10", 10"').

15. Monorail catheter (10, 10', 10", 10"') according to one of Claims 1 to 14, **characterized in that** a balloon surface (14') at least partially has a parallel and/or crisscross profiling (P) or texturing, which is preferably dimpled and/or fluted.

16. Method for operating a monorail catheter (10, 10', 10", 10"') according to one of Claims 1 to 15, **characterized in that** the balloon (14) or several balloons (14) of the same diameter are subjected to 0.5 to 2 bar.

17. Method for operating a monorail catheter (10, 10', 10", 10"') according to Claim 16, **characterized in that** the balloon (14) is preferably subjected to 1 bar.

18. Method for operating the monorail catheter (10, 10', 10", 10"') according to Claim 16 or 17, **characterized in that** the same balloon (14) can seal off human and/or animal organs, preferably coronary vessels (20), in a diameter range of preferably 1.5 to 5.5 mm.

## Revendications

1. Cathéter monorail (10, 10', 10", 10"') comprenant une extrémité de cathéter intégrée (12) ou une extrémité de cathéter rapportée (30), cathéter monorail (10, 10', 10", 10"') étant réalisé en une couche ou en plusieurs couches sous la forme d'un cathéter à ballonnet en liaison avec un ballonnet (14) et comportant au moins une lumière de fil de guidage (16) et une lumière d'alimentation/de purge (22) destinée à remplir et vider le ballonnet, et le cathéter monorail (10, 10', 10", 10"') comprenant au moins une lumière libre supplémentaire (18) destinée à l'introduction d'un milieu liquide et/ou gazeux, **caractérisé en ce qu'**une lumière la plus extérieure ou une tige extérieure (22) comporte à une extrémité avant, située à l'intérieur du ballonnet (14), un biseau (S") effilé vers l'avant.

2. Cathéter monorail (10, 10', 10", 10"') selon la revendication 1, **caractérisé en ce qu'**une couche externe (34) comprend un matériau flexible, mais seulement légèrement extensible, de préférence en polyamide, nylon et/ou polyester.

3. Cathéter monorail (10, 10', 10", 10"') selon la revendication 1 ou 2, **caractérisé en ce que** l'ouverture de sortie de la lumière libre (18) a, dans la région de l'extrémité de cathéter (12, 30), un diamètre de 0,05 à 0,45 mm, en particulier d'environ 0,35 mm.

4. Cathéter monorail (10, 10', 10", 10"') selon l'une des revendications 1 à 3, **caractérisé en ce qu'**il est conçu avec une section transversale partiellement constante ou variable, circulaire, ovale et/ou elliptique sur l'axe longitudinal (x-x) du cathéter.

5. Cathéter monorail (10, 10', 10", 10"') selon l'une des revendications 1 à 4, **caractérisé en ce qu'**au moins une couche du cathéter monorail (10, 10', 10", 10"') et/ou le fil de guidage est au moins partiellement coloré, réfléchissant et/ou fluorescent, et ou un marqueur radio-opaque est appliqué, de préférence un anneau marqueur radio-opaque.

6. Cathéter monorail (10, 10', 10", 10"') selon la revendication 5, **caractérisé en ce que** le marqueur radio-opaque est monté à l'intérieur de l'une desdites lumières (16, 18, 22) du cathéter (10, 10', 10", 10"').

7. Cathéter monorail (10, 10', 10", 10"') selon la revendication 6, **caractérisé en ce qu'**une autre lumière s'étend à l'intérieur de la lumière qui contient le marqueur radio-opaque, le marqueur radio-opaque étant fixé, en particulier par serrage, de préférence à cette autre lumière.

8. Cathéter monorail (10, 10', 10", 10"') selon l'une des revendications 6 à 7, **caractérisé en ce que** la limite extérieure du marqueur radio-opaque a, perpendiculairement à la direction longitudinale de la lumière, une section transversale ovale ou elliptique plus petite qu'une section transversale intérieure de la lumière contenant le marqueur radio-opaque.

9. Cathéter monorail (10, 10', 10", 10"') selon la revendication 1, **caractérisé en ce que** la lumière la plus extérieure ou la tige extérieure (22) est formée à la manière d'un filetage à l'extrémité avant dans une région d'enveloppe (22.1, 22.2).

10. Cathéter monorail (10, 10', 10", 10"') selon l'une des revendications 1 à 9, **caractérisé en ce qu'**il est conçu pour s'étendre obliquement vers l'avant dans la région de l'extrémité de cathéter (12, 30), un diamètre extérieur (D_{AV}) du côté avant (29, 42) de l'extrémité de cathéter (12, 30) étant de préférence de 0,35 à 0,5 mm, en particulier d'environ 0,45 mm, et/ou un diamètre extérieur (D_{AR}) du côté arrière (29', 38) de l'extrémité de cathéter (12, 30) étant de préférence de 0,45 à 0,6 mm, en particulier d'environ 0,5 mm.

11. Cathéter monorail (10, 10', 10", 10"') selon l'une des revendications 1 à 10, **caractérisé en ce que** l'extrémité de cathéter (12, 30) a une longueur (L) de 0,5 à 10 mm, en particulier de 3 à 5 mm et/ou le cathéter monorail (10, 10', 10", 10"') a une longueur totale de 800 à 2000 mm, en particulier de 1200 à 1600 mm.

12. Cathéter monorail (10, 10', 10", 10"') selon l'une des revendications 1 à 11, **caractérisé en ce qu'**une zone de liaison (40) est fondue pour former une extrémité de cathéter rapportée (30) et/ou un biseau (S) de l'extrémité de cathéter intégrée (12) ou de l'extrémité de cathéter rapportée (30), de préférence à l'une des extrémités (E, E'), ou aux deux, de l'extrémité de cathéter (12, 30).

13. Cathéter monorail (10, 10', 10", 10"') selon la revendication 12, **caractérisé en ce que**, dans la zone de liaison (40) et/ou au moins à une extrémité du ballonnet, le cathéter monorail (10, 10', 10", 10"') est réalisé de préférence en une seule couche souple.

14. Cathéter monorail (10, 10', 10", 10"') selon la revendication 12 ou 13, **caractérisé en ce que**, dans la zone de liaison (40) du cathéter monorail (10, 10', 10", 10"'), l'extrémité de cathéter (12, 30) est formé de manière à pouvoir être relié à la face d'extrémité du cathéter monorail (10, 10', 10", 10"') dans l'embout de cathéter (12, 30) et/ou l'extrémité de cathéter (12, 30) peut être insérée dans le cathéter monorail (10, 10', 10", 10'").

15. Cathéter monorail (10, 10', 10", 10"') selon l'une des revendications 1 à 14, **caractérisé en ce qu'**une surface de ballonnet (14') présente au moins partiellement un profilage (P) ou une texturation parallèle et/ou transversal qui est de préférence nopé et/ou rainuré.

16. Procédé de fonctionnement du cathéter monorail (10, 10', 10", 10'") selon l'une des revendications 1 à 15, **caractérisé en ce que** le ballonnet (14) ou plusieurs ballons (14) de même diamètre sont mis à une pression de 0,5 à 2 bars.

17. Procédé de fonctionnement du cathéter monorail (10, 10', 10", 10"') selon la revendication 16, **caractérisé en ce que** le ballonnet (14) est de préférence mis à une pression de 1 bar.

18. Procédé de fonctionnement du cathéter monorail (10, 10', 10", 10'") selon la revendication 16 ou 17, **caractérisé en ce que** le même ballonnet (14) peut obturer des organes humains et/ou animaux, de préférence des vaisseaux coronaires (20), dans une game de diamètres allant de préférence de 1,5 à 5,5 mm.
